# EUROPÄISCHE PATENTSCHRIFT

(11) **EP 0 492 254 B1**
(45) Veröffentlichungstag und Bekanntmachung des Hinweises auf die Patenterteilung: **17.03.1999**
(21) Anmeldenummer: 91121085.4
(22) Anmeldetag: 09.12.1991
(51) Int. Cl.: C07D 207/452

(54) **Verfahren zur Herstellung von N-tert.-Butoxycarbonyl-maleinimid**
Process for the preparation of N-(tert-butoxycarbonyl)maleimide
Procédé pour la préparation de N-(tert-butoxycarbonyl)maléimide

(30) Priorität: 20.12.1990 DE 4040999
(43) Veröffentlichungstag der Anmeldung: 01.07.1992
(73) Patentinhaber: SIEMENS AKTIENGESELLSCHAFT, 80333 München (DE)
(72) Erfinder: Kühn, Eberhard, W-8551 Hemhofen (DE); Beck, Jürgen, Dipl.-Chem., W-8520 Erlangen (DE); Ahne, Helmut, Dr., W-8551 Röttenbach (DE); Birkle, Siegfried, Dr., W-8552 Höchstadt (DE); Leuschner, Rainer, Dr., W-8520 Erlangen (DE); Sebald, Michael, Dr., W-8521 Hessdorf-Hannberg (DE); Sezi, Recai, Dr., W-8551 Röttenbach (DE); Bestmann, Hans-Jürgen, Prof., W-8520 Erlangen (DE)

(56) Entgegenhaltungen:
- EP-A- 234 327
- EP-A- 0 410 606
- EP-A- 0 523 556
- CHEMICAL ABSTRACTS, Band 83, Nr. 3, 21. Juli 1975 Columbus, Ohio, USA KELLER O. et al. "Preparation and some pro- perties of maleimido acids and maleoyl derivatives of peptides." Seite 545, Spalte 2, Zusammenfassung-Nr. 28 548x
- Chem. Ber.100 (1967), S. 421-424

## Beschreibung

Die Erfindung betrifft ein Verfahren zur Herstellung von N-tert.-Butoxycarbonyl-maleinimid.

In der EP-A-0 234 327 (bzw. in den entsprechenden US-A-4 837 124 und US-A-4 912 018) findet sich ein Hinweis auf die Verbindung N-tert.-Butoxycarbonyl-maleinimid. Dort ist ausgeführt (siehe Beispiel 22), daß diese Verbindung zusammen mit Styrol copolymerisiert wird; bei dieser Umsetzung entsteht ein N-tert.-Butoxycarbonylmaleinimid/Styrol-Copolymer (siehe dazu auch EP-A-0 292 821 bzw. die entsprechende US-A-4 775 609). Ein Herstellungsverfahren für das Monomer ist dabei aber nicht angegeben.

Aus "Helvetica Chimica Acta", Vol. 58 (1975), Seiten 531 bis 541 ist es bekannt, N-Alkyloxycarbonyl-maleinimide (Alkyl = CH₃, C₂H₅, i-C₄H₉, CH₂-C₆H₅ und CH₂-C₆H₄-NO₂-p) durch Umsetzung von Maleinimid mit den entsprechenden Alkylchlorformiaten in Gegenwart von N-Methylmorpholin herzustellen.

Aufgabe der Erfindung ist es, ein Verfahren anzugeben, mit dem die monomere Verbindung N-tert.-Butoxycarbonyl-maleinimid synthetisiert werden kann.

Dies wird erfindungsgemäß dadurch erreicht, daß Maleinimid in Gegenwart einer heterocyclischen Stickstoffverbindung mit wenigstens einem tertiären Stickstoffatom (als Base) mit einer äquimolaren Menge Di-tert.-butyl-dicarbonat oder mit einem geringen Überschuß in einem geeigneten Lösungsmittel bei Temperaturen bis zu 80°C zur Reaktion gebracht wird, wobei dem Maleinimid zunächst Di-tert.-butyl-dicarbonat und dann die Base zugesetzt wird.

In der nach dem Prioritätstag der vorliegenden Patentanmeldung veröffentlichten EP-OS 0 410 606 ist angegeben (siehe Beispiel 20), daß durch Umsetzung von Maleinimid mit Di-tert.-butyl-dicarbonat bei 0°C in Gegenwart von Kalium-tert.-butoxid N-tert.-Butoxycarbonyl-maleinimid hergestellt wird (vgl. dazu aber EP-OS 0 523 556: Spalte 2, Zeilen 9 bis 14).

Beim erfindungsgemäßen Verfahren wird Maleinimid mit etwa äquimolaren Mengen an Di-tert.-butyl-dicarbonat umgesetzt, d.h. mit äquimolaren Mengen oder mit einem geringen Überschuß. Di-tert.-butyl-dicarbonat ist ein Ester der hypothetischen Dikohlensäure mit der Formel R*-O-CO-O-CO-O-R* (mit R*= C(CH₃)₃) und dient zur Einführung der tert.-Butoxycarbonyl-Gruppe -CO-O-C(CH₃)₃ (Boc-Gruppe).

Die Umsetzung zwischen Maleinimid und Di-tert.-butyl-dicarbonat (Boc₂O) erfolgt in Gegenwart einer Base. Dabei ist wesentlich, daß dem Maleinimid zunächst Boc₂O und dann die Base zugesetzt wird. Ein weiteres wesentliches Merkmal des erfindungsgemäßen Verfahrens besteht in der Verwendung einer speziellen Base. Diese Base ist eine heterocyclische Stickstoffverbindung mit wenigstens einem tertiären Stickstoffatom, d.h. einer tertiären Aminofunktion. Dabei können auch Gemische derartiger Verbindungen als Base eingesetzt werden.

Der Stickstoffheterocyclus kann ein 5- oder 6-Ring sein, wobei auch ein polycyclischer Aufbau vorliegen kann, er kann einen aromatischen Charakter besitzen, er kann auch weitere Heteroatome, wie 0, S, P und Si, enthalten, und er kann ferner Substituenten, wie Alkyl- und Halogengruppen, tragen sowie exocyclische tertiäre Aminogruppen; primäre und sekundäre Aminogruppen beeinträchtigen dagegen die Wirksamkeit der Base. Die erforderliche Menge an Base ist abhängig von deren Struktur; sie reicht von katalytischen Mengen, beispielsweise ≤ 0,1 Gew.-%, bis hin zu äquimolaren Mengen.

Die beim erfindungsgemäßen Verfahren eingesetzte Base weist vorzugsweise eine der folgenden Strukturen auf: 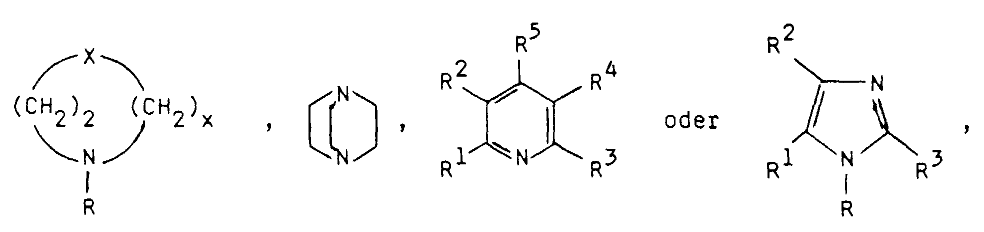 wobei folgendes gilt:
- x =: 1 oder 2;
- X =: O, S, NR, PR oder SiR₂;
- R =: Alkyl mit 1 bis 10 C-Atomen;
- R¹, R², R³ und R⁴ =: H, Alkyl mit 1 bis 10 C-Atomen, wobei R¹ und R² bzw. R³ und R⁴ cyclisch miteinander verbunden sein können, oder Halogen, insbesondere F, Cl und Br; und
- R⁵ =: H oder NR₂.

Vorteilhaft werden als Base Pyridin, 4-Dialkylaminopyridine, N-Alkyl-imidazole, N-Alkyl-morpholine und 1,4-Diazabicyclo[2.2.2]octan verwendet. Daneben kommen beispielsweise auch Chinazolin (Benzopyrimidin) und Chinoxalin (Benzopyrazin) sowie Hexamethylentetramin (1,3,5,7-Tetraazaadamantan) in Betracht.

Die Umsetzung von Maleinimid mit Boc₂O erfolgt bei Temperaturen bis zu 80°C. Vorzugsweise wird diese Reaktion bei Raumtemperatur durchgeführt, sie kann beispielsweise aber auch bei Temperaturen zwischen 50 und 80°C erfolgen. Die Reaktionsdauer beträgt > 1 min; im allgemeinen liegt die Reaktionszeit zwischen 1 und 2 h.

Die Reaktion zwischen Maleinimid und Boc₂O wird in einem Lösungsmittel durchgeführt. Vorzugsweise wird dabei ein organisches Lösungsmittel verwendet, es können aber auch wäßrige Systeme zum Einsatz gelangen, beispielsweise Dioxan/Wasser-Gemische. Als organische Lösungsmittel finden vorteilhaft Chloroform, Tetrachlorkohlenstoff, Dimethylformamid und Tetrahydrofuran Verwendung. Daneben können beispielsweise auch Methylenchlorid, Ethylacetat und N-Methylpyrrolidon zum Einsatz gelangen.

Nach der Umsetzung wird das Reaktionsgemisch - nach an sich bekannten Methoden - aufgearbeitet und das Reaktionsprodukt isoliert und gereinigt. Das N-tert.-Butoxycarbonyl-maleinimid (N-Boc-Maleinimid) wird dabei in Form farbloser Kristalle mit einem Schmelzpunkt von 62,5°C erhalten. Die Struktur dieser Verbindung wird durch die Elementaranalyse sowie durch NMR-, IR- und Massenspektren belegt.

Durch die Erfindung wird ein präparativ einfaches Syntheseverfahren für N-Boc-Maleinimid bereitgestellt, das diese Verbindung in reiner Form und in hoher Ausbeute liefert. Das monomere N-Boc-Maleinimid eignet sich besonders vorteilhaft für Polymersynthesen, insbesondere zur Herstellung von N-Boc-Maleinimid-Homopolymeren. Derartige Homopolymere sind zwar beschrieben (siehe dazu EP-A-0 292 821 bzw. die entsprechende US-A-4 775 609), ein Herstellungsverfahren für die Polymere wird aber nicht angegeben. N-tert.-Butoxycarbonyl-maleinimid kann auch zur Herstellung von Copolymeren dienen, die in Photoresists Verwendung finden; siehe dazu die EP-A-0492253 - "Photostrukturierungsverfahren".

Die Herstellung von monomeren N-Boc-Imiden, wie N-Boc-Maleinimid, ist bislang nicht bekannt; beschrieben wird lediglich die Herstellung von N-Boc-Derivaten von Lactamen und sekundären Amiden (siehe dazu: "J. Org. Chem.", Vol. 48 (1983), Seiten 2424 bis 2426) sowie von N-Boc-Pyrrolen (siehe dazu: "Angew. Chem.", Bd. 96 (1984), Seiten 291 und 292). Hierbei wird das System Triethylamin/4-Dimethylaminopyridin (NEt₃/DMAP) als Base eingesetzt. Dieses System ist beim erfindungsgemäßen Verfahren aber ungeeignet. Es hat sich nämlich gezeigt, daß N-Boc-Maleinimid durch Umsetzung von Maleinimid mit Boc₂O in Gegenwart von tertiären Aminen, wie Triethylamin und Dimethylanilin, nicht hergestellt werden kann. Die Herstellung von N-Boc-Pyrrolen kann zwar auch ohne Triethylamin erfolgen; hierbei wird zu einer Lösung eines Pyrrols in wasserfreiem Acetonitril DMAP und Boc₂O gegeben. Diese Vorgehensweise führt aber bei N-Boc-Maleinimid nicht zum Erfolg, vielmehr erfolgt hierbei eine Polymerisation des Maleinimids.

Anhand von Ausführungsbeispielen (zur Herstellung von N-Boc-Maleinimid) soll die Erfindung noch näher erläutert werden.

### Beispiel 1

10 g Maleinimid (0,1 mol) und 24 g Di-tert.-butyl-dicarbonat (0,11 mol) werden unter Argon als Schutzgas in 100 ml Chloroform vorgelegt. Zu dieser Lösung werden 78,5 mg 4-Dimethylaminopyridin (0,001 mol) gegeben, dann wird das Gemisch 4 h bei Raumtemperatur gerührt. Anschließend wird je zweimal mit Wasser und Natriumhydrogencarbonatlösung und nochmals mit Wasser ausgeschüttelt, und dann werden die vereinigten organischen Phasen über Magnesiumsulfat getrocknet. Nachfolgend wird das Lösungsmittel entfernt und der Rückstand in heißem Petrolether (60 bis 80°C) aufgeschlämmt. Das durch Abkühlen der Aufschlämmung (im Eisbad) ausfallende Kristallisat wird abgesaugt und getrocknet. Das dabei erhaltene Gemisch wird sublimiert (75°C/3x0,02 Torr), wobei farbloses N-Boc-Maleinimid erhalten wird; Ausbeute: 16,8 g (82,8 % der Theorie); Mr = 197,2 g/mol; Schmp.: 62,5°C.

### Beispiel 2

10 g Maleinimid (0,1 mol) und 24 g Di-tert.-butyl-dicarbonat (0,11 mol) werden unter Argon als Schutzgas in 100 ml Dimethylformamid vorgelegt. Zu dieser Lösung werden 78,5 mg 4-Dimethylaminopyridin (0,001 mol) gegeben, dann wird das Gemisch 1 h bei Raumtemperatur gerührt. Anschließend wird die Lösung eingeengt und mit dem 2,5fachen Volumen an Petrolether versetzt; dabei fällt das N-Boc-Maleinimid aus. Das Produkt wird abfiltriert, getrocknet und sublimiert; Ausbeute: 17,2 g (85 % der Theorie); Schmp.: 62,5°C.

### Beispiel 3

10 g Maleinimid (0,1 mol) und 22 g Di-tert.-butyl-dicarbonat (0,1 mol) werden unter Argon als Schutzgas in 100 ml Tetrahydrofuran vorgelegt. Zu dieser Lösung werden 8 ml Pyridin (0,1 mol) gegeben, dann wird das Gemisch 24 h bei Raumtemperatur gerührt. Anschließend werden die flüchtigen Komponenten am Rotationsverdampfer (bei 35°C) und im Wasserstrahlvakuum entfernt. Der Rückstand wird mit 200 ml Wasser versetzt und dreimal mit je 100 ml Methylenchlorid ausgeschüttelt. Die vereinigten organischen Phasen werden dann noch mit je 100 ml Wasser, 5 %iger Natriumhydrogencarbonatlösung und nochmals mit Wasser gewaschen. Nach dem Trocknen der organischen Phase mit Natriumsulfat wird das Lösungsmittel entfernt. Der braune Rückstand wird in 100 ml heißem Petrolether (60 bis 80°C) aufgeschlämmt. Das durch Abkühlen der Aufschlämmung (im Eisbad) ausfallende Kristallisat wird abgesaugt und getrocknet. Das dabei erhaltene Gemisch wird sublimiert (75°C/3x0,02 Torr), wobei farbloses N-Boc-Maleinimid erhalten wird; Ausbeute: 18,8 g (92,8 % der Theorie); Mr = 197,2 g/mol; Schmp.: 62,5°C. Die spektroskopischen Daten (¹H-NMR, ¹³C-NMR, IR, UV und MS) bestätigen die Struktur.

### Beispiel 4

1 g Maleinimid (0,01 mol), 2,2 g Di-tert.-butyl-dicarbonat (0,01 mol), 1,2 g N-Ethylmorpholin (0,01 mol) und 10 ml Tetrachlorkohlenstoff werden analog den Beispielen 1 bis 3 miteinander vermischt, dann wird 6 h auf eine Temperatur von 60°C erwärmt; das Maleinimid geht dabei in Lösung. Nach dem Abkühlen wird die Lösung mit Wasser ausgeschüttelt und dann eingeengt. Das dabei erhaltene Kristallisat wird isoliert und aus Petrolether umkristallisiert; Ausbeute: 1,61 g (81,5 % der Theorie); Schmp.: 62,5°C.

## Patentansprüche

1. Verfahren zur Herstellung von N-tert.-Hutoxycarbonylmaleinimid, **dadurch gekennzeichnet**, daß Maleinimid in Gegenwart einer heterocyclischen Stickstoffverbindung mit wenigstens einem tertiären Stickstoffatom (als Base) mit einer äquimolaren Menge Di-tert.-butyl-dicarbonat oder mit einem geringen Überschuß in einem geeigneten Lösungsmittel bei Temperaturen bis zu 80°C zur Reaktion gebracht wird, wobei dem Maleinimid zunächst Di-tert.-butyl-dicarbonat und dann die Base zugesetzt wird.

2. Verfahren nach Anspruch 1, **dadurch gekennzeichnet**, daß als Base eine der folgenden Verbindungen eingesetzt wird: 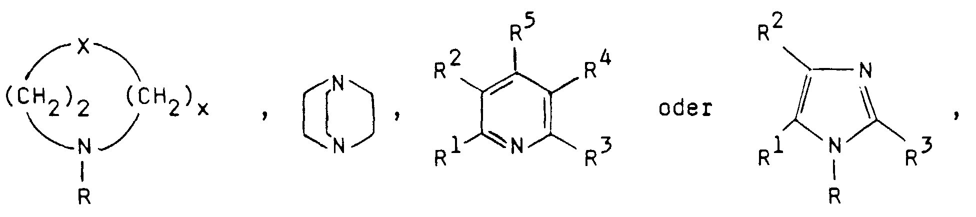 wobei folgendes gilt:
x = 1 oder 2;
X = O, S, NR, PR oder SiR₂;
R = Alkyl mit 1 bis 10 C-Atomen;
R¹, R², R³ und R⁴ = H, Alkyl mit 1 bis 10 C-Atomen, wobei R¹ und R² bzw. R³ und R⁴ cyclisch miteinander verbunden sein können, oder Halogen; und
R⁵ = H oder NR₂.

3. Verfahren nach Anspruch 2, **dadurch gekennzeichnet**, daß als Base Pyridin, 4-Dialkylaminopyridin, N-Alkyl-imidazol, N-Alkyl-morpholin oder 1,4-Diazabicyclo[2.2.2]octan verwendet wird.

4. Verfahren nach einem der Ansprüche 1 bis 3, **dadurch** **gekennzeichnet**, daß die Reaktion bei Raumtemperatur durchgeführt wird.

5. Verfahren nach einem oder mehreren der Ansprüche 1 bis 4, **dadurch gekennzeichnet**, daß ein organisches Lösungsmittel verwendet wird.

6. Verfahren nach Anspruch 5, **dadurch gekennzeichnet**, daß als Lösungsmittel Chloroform, Tetrachlorkohlenstoff, Dimethylformamid oder Tetrahydrofuran verwendet wird.

## Claims

1. Method for the preparation of N-tert.-butoxycarbonyl-maleinimide, characterised in that maleinimide is reacted in the presence of a heterocyclic nitrogen compound having at least one tertiary nitrogen atom (as a base) with an equimolar amount of di-tert.-butyl-dicarbonate or with a small excess thereof in a suitable solvent at temperatures of up to 80°C, with there being added to the maleinimide first di-tert.-butyl-dicarbonate and then the base.

2. Method according to claim 1, characterised in that one of the following compounds is used as a base: 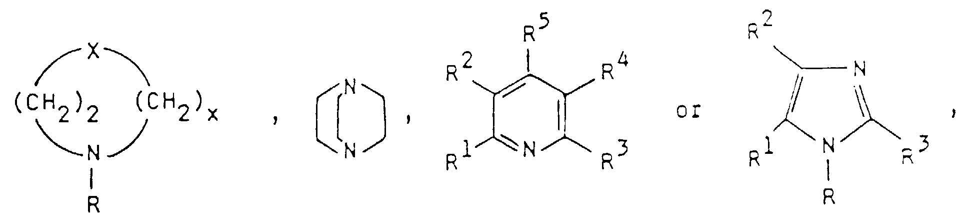 where the following applies:
x = 1 or 2;
X = O, S, NR, PR or SiR₂;
R = alkyl with 1 to 10 C atoms;
R¹, R², R³ and R⁴ = H, alkyl with 1 to 10 C atoms,
where R¹ and R² or R³ and R⁴ can be cyclically linked to each other, or halogen; and
R⁵ = H or NR₂.

3. Method according to claim 2, characterised in that pyridine, 4-dialkylaminopyridine, N-alkylimidazole, N-alkyl-morpholine or 1,4-diazabicyclo[2.2.2]octane is used as a base.

4. Method according to one of the claims 1 to 3, characterised in that the reaction is carried out at room temperature.

5. Method according to one or more of the claims 1 to 4, characterised in that an organic solvent is used.

6. Method according to claim 5, characterised in that chloroform, carbon tetrachloride, dimethyl formamide or tetrahydrofuran is used as said solvent.

## Revendications

1. Procédé pour la préparation du N-tert.-butoxycarbonylmaléimide, caractérisé en ce qu'on amène à réagir du maléimide en présence d'un composé azoté hétérocyclique contenant au moins un atome d'azote tertiaire (à titre de base) avec une quantité équimolaire de di-tert.-butyldicarbonate ou avec un léger excès dans un solvant approprié à des températures s'élevant jusqu'à 80°C, dans lequel on ajoute au maléimide d'abord le di-tert.-butyl-dicarbonate, puis la base.

2. Procédé selon la revendication 1, caractérisé en ce qu'on met en oeuvre, à titre de base, un des composés ci-après: 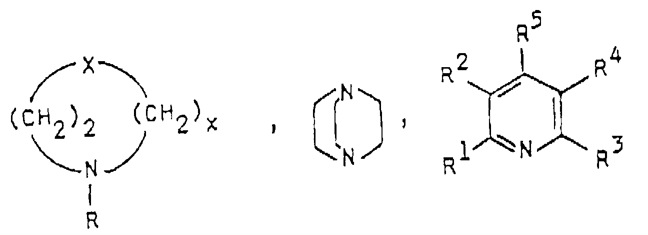 ou 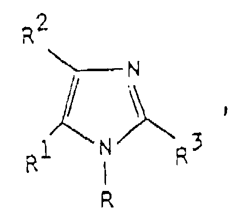 dans lesquels
x représente 1 ou 2;
X représente un atome d'oxygène, un atome de soufre, un groupe NR, un groupe PR ou un groupe SiR₂;
R représente un groupe alkyle contenant de 1 à 10 atomes de carbone,
R¹, R², R³ et R⁴ représentent un atome d'hydrogène, un groupe alkyle contenant de 1 à 10 atomes de carbone, R¹ et R², respectivement R³ et R⁴ pouvant être reliés l'un à l'autre pour former un cycle, ou représentent un atome d'halogène; et
R⁵ représente un atome d'hydrogène ou un groupe NR₂.

3. Procédé selon la revendication 2, caractérisé en ce qu'on utilise, à titre de base, de la pyridine, une 4-dialkylaminopyridine, un N-alkyl-imidazole, une N-alkylmorpholine ou le 1,4-diazabicyclo[2.2.2]octane.

4. Procédé selon l'une quelconque des revendications 1 à 3, caractérisé en ce qu'on effectue la réaction à la température ambiante.

5. Procédé selon une ou plusieurs des revendications 1 à 4, caractérisé en ce qu'on utilise un solvant organique.

6. Procédé selon la revendication 5, caractérisé en ce qu'on utilise, à titre de solvant, le chloroforme, le tétrachlorure de carbone, le diméthylformamide ou le tétrahydrofurane.
